(19) **Europäisches Patentamt / European Patent Office / Office européen des brevets**

(11) **EP 1 118 350 B1**

(12) **EUROPÄISCHE PATENTSCHRIFT**

(45) Veröffentlichungstag und Bekanntmachung des Hinweises auf die Patenterteilung:
**03.09.2008 Patentblatt 2008/36**

(51) Int Cl.:
*A61N 1/362* (2006.01)   *A61N 1/39* (2006.01)
*A61B 5/046* (2006.01)

(21) Anmeldenummer: **01100556.8**

(22) Anmeldetag: **10.01.2001**

(54) **Verfahren zur prädiktionalen Berechnung eines kardiologischen Signalverlaufs und zur Steuerung der Stimulationsabgabe eines kardiologischen Geräteimplantates**

Method for predicting a cardiac signal form and for controlling the delivery of stimulation in an implantable cardiac device

Procédé de prédiction d'une forme d'un signal cardiaque et de commande d'administration de stimulation dans un appareil cardiaque implantable

(84) Benannte Vertragsstaaten:
**AT BE CH CY DE DK ES FI FR GB GR IE IT LI LU MC NL PT SE TR**

(30) Priorität: **19.01.2000 DE 10001890**

(43) Veröffentlichungstag der Anmeldung:
**25.07.2001 Patentblatt 2001/30**

(73) Patentinhaber: **BIOTRONIK GmbH & Co. KG 12359 Berlin (DE)**

(72) Erfinder: **Meyer, Wolfgang 91052 Erlangen (DE)**

(74) Vertreter: **Lindner-Vogt, Karin L. BIOTRONIK GmbH & Co. KG Corporate Intellectual Properties Woermannkehre 1 12359 Berlin (DE)**

(56) Entgegenhaltungen:
WO-A-98/22183        US-A- 5 476 503

- THRONE R ET AL: "AUTOREGRESSIVE MODELING OF EPICARDIAL ELECTROGRAMS DURING VENTRICULAR FIBRILLATION" IEEE TRANSACTIONS ON BIOMEDICAL ENGINEERING, IEEE INC. NEW YORK, US, Bd. 40, Nr. 4, 1. April 1993 (1993-04-01), Seiten 379-386, XP000413431 ISSN: 0018-9294
- BAYKAL A ET AL: "ESTIMATION OF THE VENTRICULAR FIBRILLATION DURATION BY AUTOREGRESSIVE MODELING" IEEE TRANSACTIONS ON BIOMEDICAL ENGINEERING, IEEE INC. NEW YORK, US, Bd. 44, Nr. 5, 1. Mai 1997 (1997-05-01), Seiten 349-356, XP000658767 ISSN: 0018-9294

**Beschreibung**

Die Erfindung betrifft ein Verfahren zur prädiktionalen Berechnung eines kardiologischen Signalverlaufs und ein darauf aufbauendes Steuerungsverfahren für die Stimulationsimpulsabgabe eines kardiologischen Implantates.

Zum Hintergrund der Erfindung ist festzuhalten, daß verschiedene pathologische Zustände des Herzens durch sich signifikant ausbreitende Wellenfronten der elektrischen Erregung des Myokard charakterisiert sind. So wird das Auftreten eines sogenannten Vorhofflimmerns - einer atrialen Fibrillation (im folgenden: AF) - durch die Propagation von Wellenfronten auf dem atrialen Myokard erklärt. Diese Wellenausbreitung spiegelt sich im lokal an einer bipolaren Elektrode gemessenen Potential wieder.

Gemäß einschlägiger medizinischer Erkenntnisse ist es bekannt, mit Hilfe einer Elektrode während der sogenannten "erregbaren Lücke" - also der kurzen Zeitspanne vor dem Eintreffen einer neuen, einen Fibrillationszustand charakterisierenden Wellenfront am Ort der Elektrode und nach Ablauf der lokalen Refraktärzeit - einen Stimulationsimpuls über die Elektrode zu applizieren, der eine Art "Reset" für den ganzen Herzmuskelapparat bewirkt und die Fibrillation unterbindet. Das Verfahren ist jedoch von herkömmlichen Defibrillationsverfahren zu unterscheiden, die erst auf das Eintreffen der Wellenfront reagieren können, da es weniger Defibrillationsenergie benötigt und Nebenwirkungen wie das Auslösen von Kammer-Tachykardien oder postdefibrillatorischer Bradykardie vermeidet.

Ein Verfahren nach dem Oberbegriff des Anspruchs 1 ist aus Throne R et al: "Autoregressive modeling of Epicardial Electrograms during Ventricular fibrillation", IEEE Transaction Biomedical Engineering, IEEE INC. New York, US. Bd 40, Nr 4,1. April 1993, Seiten 379-386 bekannt.

Zur Bekämpfung tachykarder Zustände des Herzens sind nun bereits verschiedene Ansätze aus dem Stand der Technik bekannt geworden. So beschäftigt sich die EP 0 830 876 A1 mit einer implantierbaren Vorrichtung zur Tachykardie-Früherkennung und -Unterdrückung beim Herzen, bei der eine in Kontakt mit dem Herzmuskelgewebe stehende Mikroelektroden-Anordnung die Reizleitungspotentiale im Herzmuskelgewebe erfaßt. Eine Meßeinrichtung bestimmt die Refraktärzeit der Herzmuskelzellen im überwachten Herzbereich und die Reizleitungsgeschwindigkeit im überwachten Herzmuskelbereich. Der Produktwert aus Refraktärzeit und Reizleitungsgeschwindigkeit ist repräsentativ für das Tachykardie-Risiko. Unterschreitet der Produktwert einen bestimmten Tachykardie-Grenzwert, so signalisiert dies einen Tachykardie-gefährdeten Zustand des Herzens. Ist ein solcher Zustand erkannt, kann mit Hilfe einer Stimulationsvorrichtung ein antitachykarder Stimulationsimpuls abgegeben werden.

Von Nachteil bei diesem Stand der Technik ist die Tatsache, daß das dort offenbarte kardiologische Geräteimplantat wegen der vorzusehenden Mikroelektroden-Anordnung nicht einfach durch entsprechende steuerungstechnische Auslegung eines in seiner äußeren Ausprägung ansonsten üblichen Herzschrittmachers oder Defibrillators realisierbar ist.

Um nun mit einer herkömmlichen Implantatanordnung arbeiten zu können, sind Wege zu finden, mittels denen die erregbare Lücke im Atrium bestimmbar und der lokale Potentialverlauf am Ort der Elektrode vorhersagbar ist. Dabei ist zu beachten, daß die gesamte räumliche und zeitliche Verteilung der lokalen Potentiale über das Atrium oder Informationen über die inneren Freiheitsgrade der Myokardzellen nicht zur Verfügung stehen.

Als Lösungsansatz für die vorstehende Problematik schlägt die Erfindung nun ein Verfahren zur prädiktionalen Berechnung eines kardiologischen Signalverlaufs vor, dessen wesentliche Verfahrensschritte in Anspruch 1 angegeben sind:

- Abtasten des zeitlichen Verlaufs eines kardiologischen Signals während einer bestimmten Abtastperiode,
- Berechnung von Modellkoeffizienten nach der Methode der autoregressiven Modellierung aus den Abtastwerten der Abtastperiode,
- prädiktionale Berechnung von der Abtastperiode folgenden, zu erwartenden Signalwerten während einer Prädiktionsperiode auf der Basis der Abtastwerte und der Modellkoeffizienten nach der Methode der autoregressiven Modellierung, wobei die Signalwerte iterativ jeweils durch Einsetzen des zuletzt vorausberechneten Signalwertes als letzter Abtastwert bei der autoregressiven Modellierung berechnet werden.

Die Erfindung geht dabei von der Erkenntnis aus, daß es sich bei der atrialen Fibrillation als Beispiel für einen pathologischen Zustand des Herzens um einen irregulären Prozeß handelt, der an einer lokalen Elektrode, wie sie bei Herzschrittmachern oder Defibrillatoren Verwendung findet, teilweise als unregelmäßiger Potentialverlauf, teilweise in Form geordneter Potentialstrukturen auftritt. Diese beobachteten Unregelmäßigkeiten werfen die Frage auf, ob eine atriale Fibrillation aufgrund der zahlreichen Nicht-Linearitäten in biologischen Systemen als chaotisch zu gelten hat, so daß entsprechende Analyse- und Kontrollmethoden anzuwenden wären. Bislang allerdings ist das Auftreten chaotischer Verhältnisse in Fibrillationsepisoden unbewiesen. Vielmehr haben Vorversuche bei der Entwicklung des anmeldungsgemäßen Verfahrens z.B. mit Hilfe der nicht-linearen Approximation von Meßsignalen und Bestimmung der sogenannten Lyapunov-Exponenten sowie auch eine qualitative Betrachtung von Meßsignalen keine zwingenden Erkenntnisse für das Vorhandensein von deterministischer exponentieller Divergenz der Fibrillationspotentiale ergeben. Insbesondere sind während akuter atrialer Fibrillation häufige Übergänge zwischen periodischen Abschnitten und irregulären Intervallen festzustellen. Dies deutet darauf hin, daß sowohl eine starke lineare Komponente, wie auch stochastische Einflüsse bei AF-Potentialverlauf und -ausbreitung vorhanden sind. Auch wenn nun in diesem Zusammenhang die künftige Identifizierung chaotischer Beiträge, die dann entsprechend der Erfindung mit einem nicht-linearen autoregressiven Modell prädiktional berechnet werden könnten, zur atrialen Fibrillation nicht auszuschließen ist, so ist beim gegenwärtigen Stand der Forschung aus der Spekulation über chaotisches Verhalten keinerlei Verfahren für die Vorhersage des Potentialverlaufs während der atrialen Fibrillation bzw. für eine therapeutisch wirksame Steuerung kardiologischer Geräteimplantate im Sinne einer aktiven Beendigung des Fibrillationszustandes abzuleiten. Diese Einschränkung gilt hingegen nicht für die Prädiktion der Fibrillationsinitialisierung mit Hilfe der im wesentlichen autonom determinierten Abläufe vor dem eigentlichen Auftreten einer atrialen Fibrillation.

In diesem Sinne werden ein kardiologisches Signal, wie z.B. ein EKG-Signal, während einer bestimmten Abtastperiode abgetastet und entsprechende Meßpunkte aufgezeichnet. Nach der Methode der autoregressiven Modellierung werden aus den Abtastwerten der Abtastperiode Modellkoeffizienten berechnet und damit wiederum zu erwartende Signalwerte in Folge einer Abtastperiode während einer bestimmten Prädiktionsperiode auf der Basis der Abtastwerte und der Modellkoeffizienten nach der Methode der autoregressiven Modellierung vorausberechnet. Diese Signalwerte indizieren nun beispielsweise das Herannahen einer Erregungsfront an die Abtastelektrode, so daß zu einem physiologisch sinnvollen Zeitpunkt vor der berechneten Ankunft der Wellenfront ein antitachykarder Impuls vom Implantat abgegeben werden kann.

Die lineare autoregressive Prädiktion zur Vorausberechnung des atrialen Potentialverlaufs stellt dabei gegenüber anderen denkbaren mathematischen Methoden den vielversprechendsten Ansatz dar, da das Konzept grundsätzlich sehr einfach ist und insbesondere periodische lineare Komponenten, die in AF-Potentialen dominieren, berücksichtigt werden.

In den Unteransprüchen 2 bis 8 sind bevorzugte Verfahrensmerkmale angegeben, deren Inhalt und Bedeutung anhand eines Ausführungsbeispiels im folgenden noch näher erläutert werden.

Anspruch 9 bezieht sich auf das kardiologische Geräteimplantat als solches, das neben der üblicherweise vorhandenen Steuereinheit, Meßelektrode und Stimulationselektrode eine Recheneinheit zur prädiktionalen Berechnung eines kardiologischen Signalverlaufes nach einem der Ansprüche 1 bis 8 und eine für die elektrische Beaufschlagung der Stimulationselektrode sorgende Stimulationseinheit aufweist, die mit einem Verfahren zur Steuerung der Stimulationsimpulsabgabe eines kardiologischen Geräteimplantates, bei dem mit Hilfe des Prädiktionsverfahrens nach einem der Ansprüche 1 bis 8 ein kardiologisch anormaler Signalwertverlauf im voraus berechenbar ist und entsprechend ein diesem entgegenwirkender Stimulationsimpuls innerhalb der Prädiktionsperiode abzugeben ist betreibbar ist. Insbesondere wird das Geräteimplantat so gesteuert, daß der Stimulationsimpuls in der erregbaren Lükke vor Eintreffen einer vorausberechneten Wellenfront an der dem Impuls abgebenden Erregungselektrode bei einer atrialen Fibrillation appliziert wird

Im folgenden wird nun die Erfindung anhand der beigefügten Zeichnungen näher erläutert. Es zeigen:

Fig. 1          ein Kurvenschaubild des zeitlichen Verlaufs des vorausberechneten Signalverlaufs und des tatsächlichen Meßsignals eines kardiologischen Potentials,

Fig. 2 und 3     Histogramme des linearen Korrelationskoeffizienten zwischen vorausberechnetem Signalverlauf und Meßsignal für unterschiedliche Ordnungen der autoregressiven Modellfunktion,

Fig. 4          ein Schaubild für die Verteilungsfunktion der Diskrepanzen zwischen vorausberechnetem Signalverlauf und Meßsignal,

Fig. 5          ein Histogramm der Korrelation zwischen vorausberechnetem Signalverlauf und Meßsignal bei verminderter Abtastrate, und

Fig. 6          ein Schaubild für die Verteilungsfunktion der Diskrepanzen zwischen vorausberechnetem Signalverlauf und Meßsignal bei der Abtastrate gemäß Fig. 5.

Einleitend ist festzuhalten, daß die der nachfolgenden Beschreibung zugrunde liegenden Meßergebnisse auf Potentialsignalen beruhen, die über eine epikardial angebrachte, bipolare MAP-Elektrode bei einem Patienten nach Aortenklappenersatz abgeleitet wurden. In der zukünftigen Praxis kann ein kardiologisches Geräteimplantat, wie ein Herzschrittmacher oder Defibrillator, in der üblichen Ausprägung eine mikroprozessor-basierte Steuereinheit aufweisen, die die üblichen geräteinternen Steuervorgänge, die Verarbeitung der von außen dem Implantat zugeführten Daten und Signale und insbesondere auch die Berechnungsvorgänge zur prädiktionalen Berechnung des die propagierende AF-Wellenfront repräsentierenden Potentials im atrialen Myokard vornimmt. Zur Abtastung des Potentialverlaufs ist eine Meßelektrode vorgesehen, die getrennt von oder in Einheit mit einer Stimulationselektrode ausgebildet sein kann. Letztere kann von einer Stimulationseinheit des Geräteimplantates elektrisch beaufschlagt werden, um an das Herz einen entsprechenden Stimulationsimpuls abzugeben, der auf der Basis des vorausberechneten Signalverlaufes in der erregbaren Lükke vor Eintreffen der vorausberechneten Wellenfront appliziert wird.

Bevor nun auf das eigentliche Verfahren zur prädiktionalen Berechnung eines kardiologischen Signalverlaufs eingegangen wird, soll die der Erfindung zugrunde liegende Methode der autoregressiven Modellierung kurz dargestellt werden, wie sie von W.H. Press et al. in dem Fachbeitrag "Numerical Recipes in C", University Press, Cambridge 1992, erläutert ist.

So lautet die Grundgleichung der autoregressiven Modellierung demnach:

$$y(n) = \sum_{j=1}^{M} d(j)\, y(n-j) + x(n)$$

Sie bedeutet, daß ein künftiger, vorauszuberechnender Meßwert y(n) zum Abtastzeitpunkt n eines Signals als Linearkombination der M vorhergehenden Wert modelliert wird, wobei M Modellkoeffizienten d(j) mit j = 1 ... M auftreten und ein Vorhersagefehler x(n) in Kauf genommen wird. Die Modellkoeffizienten werden durch Bestimmung des mittleren quadratischen Vorhersagefehlers über N>=M Meßpunkte ermittelt. Sind die Koeffizienten bestimmt, so läßt sich mit Hilfe der Grundgleichung der jeweils nächste Wert vorausberechnen.

Die Erweiterung auf die Vorhersage einer größeren Zahl unbekannter Meßpunkte, also insbesondere die prädiktionale Berechnung von der eigentlichen Abtastperiode folgenden, zu erwartenden Signalwerten während einer Prädiktionsperiode auf der Basis der Abtastwerte erfolgt durch Einsetzen des ersten vorausberechneten Wertes als tatsächlichen letzten Meßwert in die oben angegebene Gleichung und Wiederholung der Vorausberechnung für diesen neuen Satz von Daten. Die Signalwerte werden also iterativ jeweils durch Einsetzen des zuletzt vorausberechneten Signalwertes als letzter Abtastwert bei der autoregressiven Modellierung berechnet.

Grundsätzlich lassen sich die Koeffizienten für jeden Vorhersageschritt mit einem konstanten Meßfenster - also die M vorhergehenden Werte - neu bestimmen, jedoch wird dies bei den dem Ausführungsbeispiel zugrunde liegenden Untersuchungen und Berechnungen nicht vorgenommen. Vielmehr wird ein neuer Satz von Modellkoeffizienten erst mit Vorausberechnung der Signalwerte in einer neuen Prädiktionsperiode bestimmt, die Berechnung der Koeffizienten erfolgt also erst bei Verschiebung des für die Prädiktion verwendeten Fensters bekannter Meßdaten von neuem.

Die nachfolgenden Beispiele wurden mit unterschiedlichen Größen des Parameters M, also unterschiedlichen Ordnungen des autoregressiven Modells durchgeführt. Im folgenden wird anfänglich mit Modellordnungen bzw. Meßfenstern bis M=10000 gearbeitet.

Es werden zuerst mit Hilfe des Standardverfahrens nach Press et al. (a.a.O.) die Koeffizienten d(j) bestimmt. Dies erfolgt im wesentlichen durch folgende Schritte:

Bei der Vorhersage des Abtastwertes y(n) an der Stelle n mit Hilfe eines autoregressiven Modells tritt im Allgemeinen eine Abweichung x(n) der Vorhersage vom tatsächlichen Meßwert auf:

$$y(n) = \sum_{j=1}^{M} d(j)\, y(n-j) + x(n)$$

Die Koeffizienten d(j) werden daher so bestimmt, daß die mittlere quadratische Abweichung

$$\langle x(n)^2 \rangle$$

für Vorhersagen innerhalb des bekannten und als Grundlage der Prädiktion dienenden Datensatzes der Länge N minimal wird. Mit der bekannten Autokorrelationsfunktion Φ(j) der bekannten Abtastwerte

$$\Phi(j) \equiv (N-j)^{-1} \sum_{i=1}^{N-j} y(i)\, y(i+j)$$

ergibt sich aus der Forderung nach Minimierung des Vorhersagefehlers x(n) ein System aus M linearen Gleichungen für die gesuchten Größen d(j):

$$\sum_{j=1}^{M} \Phi(|j-k|)\, d(j) = \Phi(k)$$

Die Auflösung dieses Systems nach d(j) und damit die Bestimmung der Parameter des autoregressiven Modells erfolgt durch Triangulation der dem linearen Gleichungssystem zugeordneten Koeffizientenmatrix, z.B. mit Hilfe des Eliminationsverfahrens nach Gauss-Jordan.

Ferner ist als weiterer Parameter die Länge des vorherzuberechnenden Signalverlaufs festzulegen. Bei den folgenden Beispielsrechnungen wird diese bei p=100 konstant gehalten. Die Berechnungen erfolgen dann jeweils anhand eines Fensters, das die Anzahl der Meßpunkte N=M+1 enthält. Nach prädiktionaler Vorausberechnung von 100 Meßpunkten mit festen Modellkoeffizienten wird das Fenster um einen Punkt verschoben. Danach werden die Modellkoeffizienten erneut angepaßt und berechnet und eine neue prädiktionale Berechnung durchgeführt.

Umgesetzt in Größen der praktischen Messung bedeuten bei einer Abtastrate des Erregungspotentials des Herzens mit 500 Hz die Länge des vorherzuberechnenden Signals von p=100 eine Prädiktionsperiode von 200 ms. Die Modellordnung bzw. das Meßfenster der Länge M=10000 entspricht folglich einer Abtastperiode von 20 s. Erfolgt die Verschiebung des Meßfensters in 5000 Schritten, so erfolgt die prädiktionale Berechnung aus Abtastwerten in Form von Meßdaten über 20 Sekunden hinweg in eine in der Zukunft liegende Prädiktionsperiode von 200 ms, wobei für einen Zeitraum von 10 Sekunden von jedem Abtastschritt die gesamte Vorausberechnung durchgeführt wird.

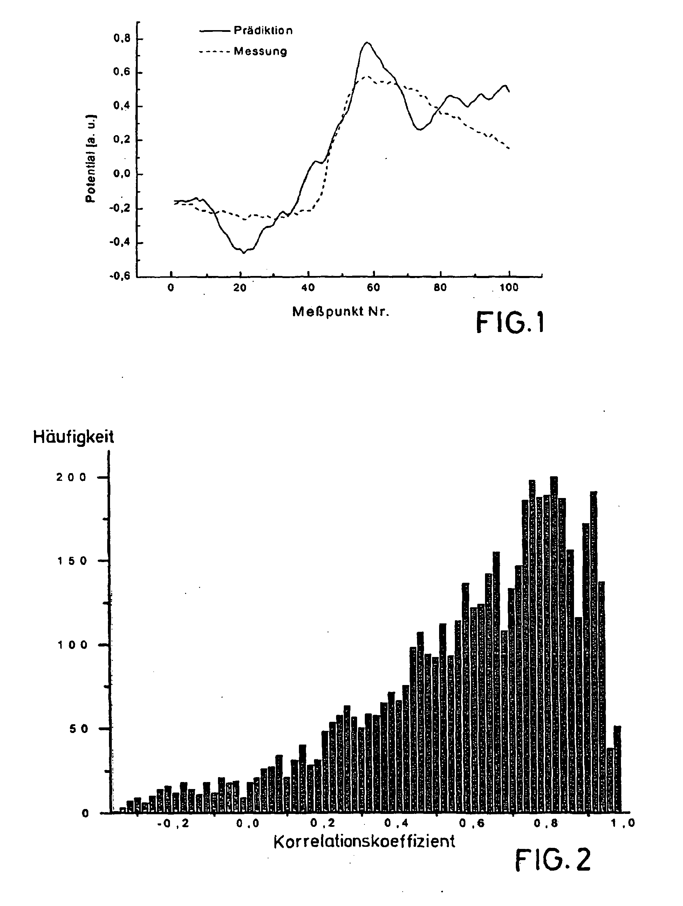

Wird dies anhand einer praktischen Messung vollzogen, so ergeben sich die in Fig. 1 mit einer durchgezogenen Linie aufgetragenen, zu erwartenden Signalwerte. Fig. 1 zeigt einen typischen Verlauf des prädiktional vorausberechneten Signals in einem Fenster der Länge 200 ms mit M=10000.

Zu Kontrollzwecken wurde mit der eingangs dargelegten Meßanordnung auch der tatsächliche Verlauf des Potentialsignals für das Meßfenster aufgezeichnet. Es ergibt sich der in Fig. 1 strichliert dargestellte Kurvenverlauf. Dieser weist zwar im Vergleich zum vorausberechneten Kurvenverlauf (durchgezogene Linie) Unterschiede im Detail auf, jedoch ist mit hoher Signifikanz eine globale Übereinstimmung zwischen Hauptkriterien, wie den Lagen der ansteigenden Flanke und des Maximums des Potentialsignals, festzustellen.

Wird nun in einem kardiologischen Geräteimplantat aufgrund der dort zur Verfügung stehenden Meßdaten mit dem erfindungsgemäßen Verfahren der zu erwartende Signalverlauf in der beschriebenen Weise ständig vorausberechnet und es ergibt sich für eine bestimmte Prädiktionsperiode ein Signalverlauf gemäß Fig. 1, so "weiß" das Geräteimplantat mit hoher Wahrscheinlichkeit, daß eine AF-Wellenfront auf seine Meßelektrode zuläuft. Da es diesen kardiologisch anormalen Signalwertverlauf vorausberechnet, kann ein diesem anormalen Signalverlauf und damit der propagierenden Wellenfront entgegenwirkender Stimulationsimpuls innerhalb der Prädiktionsperiode in der sogenannten "erregbaren Lücke" an das Herz abgegeben werden, so daß die Wellenfront unterbrochen und eine Fibrillation beendet werden kann.

Um nun die Qualität des vorstehend erörterten Vorausberechnungsverfahrens zu beurteilen, wurden auf der Basis von 5000 Verschiebungen des Meßfensters insgesamt 5000 Vorausberechnungen und deren Korrelation mit dem gemessenen Signalverlauf zugrunde gelegt und für jede Vorausberechnung der lineare Korrelations-Koeffizient bestimmt. Mit üblichen statistischen Auswertemethoden wurden statistische Parameter der Verteilung der Korrelation für 5000 Vorausberechnungen über 200 ms ermittelt, die der folgenden Tabelle entnehmbar sind:

|  | M=1000 | M=5000 | M=10000 |
|---|---|---|---|
| Mittelwert | 0,52 | 0,56 | 0,59 |
| Standardabweichung | 0,36 | 0,29 | 0,28 |

(fortgesetzt)

|  | M=1000 | M=5000 | M=10000 |
|---|---|---|---|
| Schiefe | 6,00 | 4,69 | 4,79 |
| Median | 0,62 | 0,62 | 0,66 |

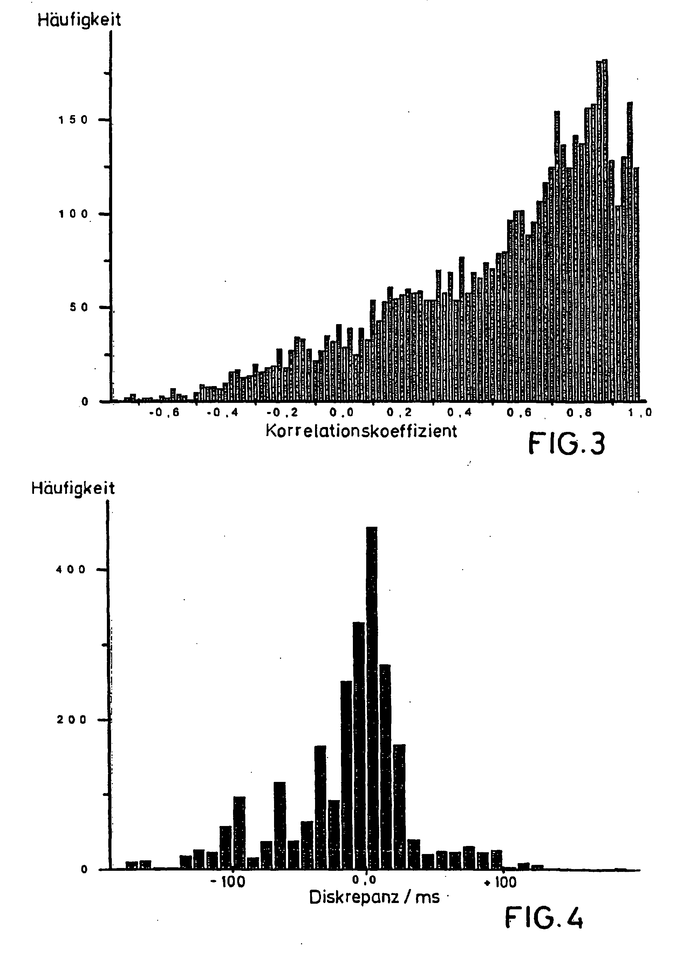

Die entsprechenden Histogramme sind in Fig. 2 und 3 für M=10000 (Fig. 2) und M=1000 (Fig. 3) dargestellt.

Zusammenfassend ergibt sich aus der Tabelle und den beiden Histogrammen in statistischer Hinsicht folgendes: Der Mittelwert nimmt mit der Ordnung des Modells zu. Der Median ist bei M=10000 am höchsten, was einer maximalen Anzahl hoher positiver Korrelationen entspricht. Alle Verteilungen weisen hohe Schiefe auf, wobei diese nicht mit der Ordnung M korreliert. Da die Vorhersagbarkeit ferner um so besser anzusehen ist, je weniger Koeffizienten im Bereich der Null bzw. im negativen Bereich auftreten, ist die Vorhersage bei M=10000 qualitativ am besten. Wie aus Fig. 2 im Vergleich zu Fig. 3 deutlich wird, treten bei M=10000 nämlich am meisten positive Korrelationen auf. Die negativen Korrelationen sind auf Übergänge im Signalverlauf, z.B. zwischen irregulärem und weitgehend periodischem Verhalten, zurückzuführen. Daß die absolute Zahl negativer Korrelationen deutlich von Null verschieden ist, liegt an der Verwendung eines gleitenden Fensters.

Neben der Korrelation der eigentlichen Verläufe des vorausberechneten Signals und des tatsächlichen Meßsignals ist im Rahmen der vorliegenden Erfindung auch die korrekte Vorhersage des "Aufstrichs" des Maximums von Bedeutung; was im folgenden für die bereits verwendete Ordnung M=10000 des autoregressiven Modells untersucht wird. Nach folgendem Algorithmus wird dabei untersucht, wie nahe die Vorhersage eines Potentialanstieges, der einer sich nähernden Wellenfront entspricht, dem tatsächlichen Anstieg und damit dem Eintreffen der Wellenfront kommt: Zunächst wird das Maximum des vorausberechneten Signalverlaufs gesucht. Befindet sich dieses in der linken Hälfte der Prädiktionsperiode, so wird die Vorausberechnung nicht berücksichtigt, da die Wellenfront vermutlich schon eingetroffen bzw. eine effektive Auslöschung durch Applizieren eines Gegen-Stimulationsimpulses nicht mehr möglich ist. Liegt das Maximum in der rechten Hälfte der Prädiktionsperiode, so werden die Mittelwerte von Prädiktion und tatsächlichem Meßsignal bestimmt. Der Durchlaufzeitpunkt des vorausberechneten Signalverlaufs und des tatsächlichen Meßwertes durch deren Mittelwerte wird als Beginn des "Aufstrichs" definiert. Die entsprechenden Zeitpunkte lassen sich nun vergleichen und eine Statistik ihrer Diskrepanzen aufstellen.

Das Ergebnis dieser Untersuchung ist in Fig. 4 dargestellt. Daraus ist erkennbar, daß die Verteilungsfunktion um ein deutliches Maximum um die Diskrepanz 0,0 relativ breite Ausläufer aufweist, was u.a. auf die Definition des "Aufstrichs" zurückzuführen ist. Da eine hohe Konzentration der Verteilungswerte um die Diskrepanz 0,0 vorliegt, kann mit Hilfe der einfachen Methode der autoregressiven Modellierung der Zeitpunkt des Aufstrichs in einem engen Bereich vorausberechnet werden. Eine quantitative Auswertung ergibt, daß etwa 36 % aller Vorhersagen eine Diskrepanz mit absolutem Betrag <10 ms liefern und somit in einem Fenster der Breite 20 ms um den tatsächlichen "Aufstrich" angesiedelt sind.

Nimmt man nun an, daß die "erregbare Lücke" exakt eine Breite von 20 ms aufweist, und daß der Off-Set zwischen effektiver Stimulation und Beginn der Wellenfront sowie die Breite des "Aufstrichs" abschätzbar und weitgehend konstant sind, so beträgt nach diesem Ansatz die Wahrscheinlichkeit, durch einen Extrastimulus die "erregbare Lücke" zu treffen, ein Vielfaches der Wahrscheinlichkeit bei unkoordinierter Stimulation. In letztgenannter Situation ist die Wahrscheinlichkeit, daß ein Einzelstimulus die "erregbare Lücke" trifft, nämlich gleich dem Verhältnis der Zeiten, in denen das Gewebe erregbar und in denen es nicht erregbar ist.

Da für den praktischen Einsatz des erfindungsgemäßen Verfahrens in kardiologischen Geräteimplantaten der Rechenaufwand wegen der beschränkten Kapazität solcher Geräte möglichst niedrig sein soll - was bei einer Modellordnung von M=10000 nicht der Fall ist -, wurde das erfindungsgemäße Prädiktionsverfahren auch im Hinblick auf niedrigere Modellordnungen untersucht. So wurde die Abtastrate von 500 Hz auf 50 Hz herabgesetzt. Damit wird die gleiche Zeitskala wie bei den oben erörterten Untersuchungen bereits bei 10 statt bei 100 vorausberechneten Signalwerten erreicht. Gleichzeitig nimmt die zeitliche Auflösung für die Lage des vorausberechneten "Aufstrichs" ab und beträgt ebenfalls 10 ms. Die Modellordnung reduziert sich auf M=1000.

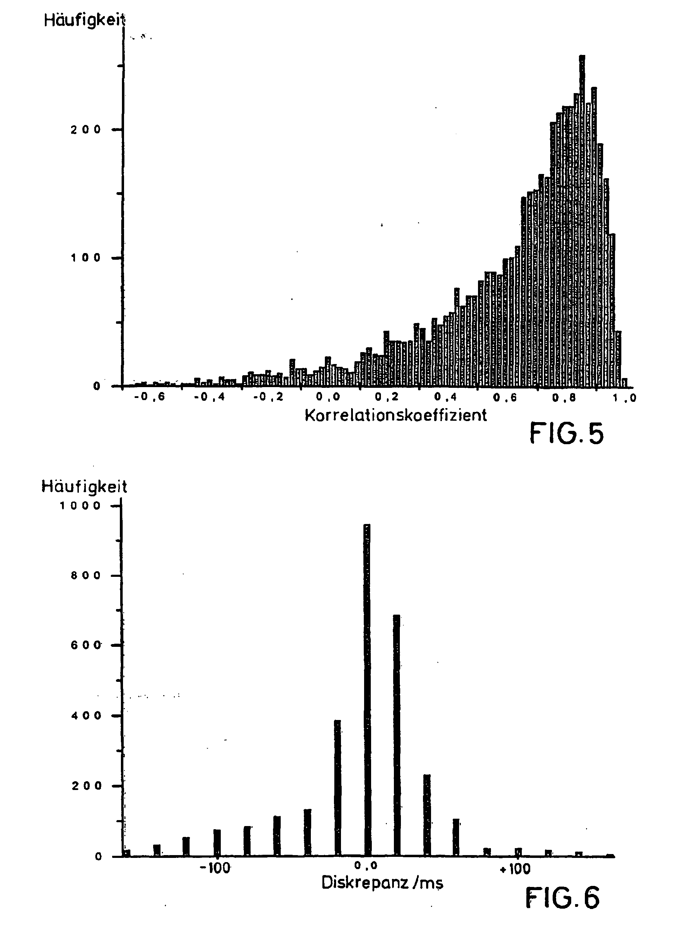

Entsprechende Berechnungen liefern das Histogramm gemäß Fig. 5 und die folgende Tabelle für statistische Parameter.

|  | M=10000 (500 Hz) | M=1000 (50 Hz) |
|---|---|---|
| Mittelwert | 0,59 | 0,63 |
| Standardabweichung | 0,28 | 0,29 |
| Schiefe | 4,79 | 5,63 |

(fortgesetzt)

|  | M=10000 (500 Hz) | M=1000 (50 Hz) |
|---|---|---|
| Median | 0,66 | 0,72 |
| Proz. Anteil im Bereich von ± 10 ms um den tatsächlichen "Aufstrich" | 36 % | 32 % |

Daraus ergibt sich, daß qualitativ die Korrelation bei verminderter Abtastrate weitgehend in Übereinstimmung mit den Ergebnissen bei einer Abtastrate von 500 Hz ist. Es liegt lediglich ein zu stärkeren negativen Korrelationen verlängerter Ausläufer der Verteilung vor. Ferner liegt der Anteil der Vorausberechnungen für den "Aufstrich", die in einem Intervall der Breite 20 ms um den tatsächlichen "Aufstrich" fallen, mit 32 % nur geringfügig unter dem Anteil von 36 % bei einer Abtastrate von 500 Hz. Damit ist die Qualität der Vorausberechnungen durch die verminderte Abtastrate nur in einem geringen Maße verschlechtert, was sich auch aus der Verteilungsfunktion der Diskrepanzen gemäß Fig. 6 ergibt. Von dem Diagramm gemäß Fig. 4 unterscheidet sich dieses lediglich in der verminderten Auflösung von lediglich 20 ms. Diskrepanzen, die zwischen +/-10 ms liegen, werden daher der Diskrepanz 0,0 ms zugeordnet.

Zusammenfassend unterscheiden sich sowohl die Verteilungen mit einer Abtastrate von 500 Hz als auch diejenigen auf der Basis der erniedrigten Abtastrate von 50 Hz, für Korrelationen und Diskrepanzen signifikant von den entsprechenden Verteilungen bei einem rein stochastischen System. Wäre der vorausberechnete globale Signalverlauf in der Prädiktionsperiode nicht mit dem tatsächlichen Signalverlauf korreliert, so wären die entsprechenden Verteilungen als symmetrische Gauß-Kurven um den Korrelations-Koeffizienten r=0,0 angeordnet. Im Falle einer Unabhängigkeit von vorhergesagtem "Aufstrich" und tatsächlichem Anstieg der Maximumsflanke wäre ferner die Verteilung der Diskrepanzen flach. Insoweit zeigen die erörterten statistischen Untersuchungen deutlich die Funktionsfähigkeit und praktische Tauglichkeit des erfindungsgemäßen Verfahrens.

**Patentansprüche**

1. Verfahren zur prädiktionalen Berechnung eines kardiologischen Signalverlaufs, mit folgenden Verfahrensschritten:

   - Abtasten des zeitlichen Verlaufs eines kardiologischen Signals während einer bestimmten Abtastperiode,
   - Berechnung von Modellkoeffizienten nach der Methode der autoregressiven Modellierung aus den Abtastwerten der Abtastperiode, **gekennzeichnet durch** eine
   - prädiktionale Berechnung von der Abtastperiode folgenden, zu erwartenden Signalwerten während einer Prädiktionsperiode auf der Basis der Abtastwerte und der Modellkoeffizienten nach der Methode der autoregressiven Modellierung, wobei die Signalwerte iterativ jeweils **durch** Einsetzen des zuletzt vorausberechneten Signalwertes als letzter Abtastwert bei der autoregressiven Modellierung berechnet werden.

2. Verfahren nach Anspruch 1, **dadurch gekennzeichnet, daß** das kardiologische Signal mittels eines kardiologischen Geräteimplantats, insbesondere eines Herzschrittmachers oder Defibrillators abgetastet wird.

3. Verfahren nach Anspruch 1 oder 2, **dadurch gekennzeichnet, daß** als kardiologisches Signal das Erregungspotential des Herzens bei der Propagation von Wellenfronten auf dem atrialen Myokard abgetastet wird.

4. Verfahren nach Anspruch 3, **dadurch gekennzeichnet, daß** das Erregungspotential mittels einer bipolaren Elektrode abgetastet wird.

5. Verfahren nach einem der Ansprüche 1 bis 4, **dadurch gekennzeichnet, daß** als Modell die autoregressive lineare Modellierung eingesetzt wird.

6. Verfahren nach Anspruch 5, **dadurch gekennzeichnet, daß** die autoregressive Modellierung nach der Grrundgleichung

$$y(n) = \sum_{j=1}^{M} d(j)\, y(n - j) + x(n)$$

mit y(n): vorberechneter Signalwert

d(j): jeweils einer von M Modellkoeffizienten und

x(n) zugelassener Vorhersagefehler

vorgenommen wird.

7.  Verfahren nach Anspruch 6, **dadurch gekennzeichnet, daß** die Modellkoeffizienten d(j) durch Bestimmung des mittleren quadratischen Vorhersagefehlers über N>=M Abtastwerte ermittelt werden.

8.  Verfahren nach einem der Ansprüche 5 bis 7, **dadurch gekennzeichnet, daß** ein neuer Satz von Modellkoeffizienten erst mit Vorausberechnung der Signalwerte in einer neuen Prädiktionsperiode bestimmt wird.

9.  Kardiologisches Geräteimplantat mit

    - einer Steuereinheit,
    - einer Messelektrode,
    - einer Recheneinheit zur prädiktionalen Berechnung eines kardiologischen Signalverlaufes nach einem der Ansprüche 1 bis 8,
    - einer Stimulationselektrode und
    - einer für die elektrische Beaufschlagung der Stimulationselektrode sorgenden Stimulationseinheit, die nach einem Verfahren zur Steuerung der Stimulationsimpulsabgabe eines kardiologischen Geräteimplantates wobei bei Vorausberechnung eines kardiologisch anormalen Signalwertverlaufes mit Hilfe des Prädiktionsverfahrens nach einem der Ansprüche 1 bis 8 ein dem anormalen Signalverlauf entgegenwirkender Stimulationsimpuls zu einem aus der Prädiktion ermittelten Zeitpunkt abgegeben wird oder einem Verfahren zur Steuerung der Stimulationsimpulsabgabe eines kardiologischen Geräteimplantates bei Vorausberechnung eines kardiologisch anormalen Signalwertverlaufes mit Hilfe des Prädiktionsverfahrens nach einem der Ansprüche 1 bis 8 ein dem anormalen Signalverlauf entgegenwirkender Stimulationsimpuls zu einem aus der Prädiktion ermittelten Zeitpunkt abgegeben wird wobei der Stimulationsimpuls in der erregbaren Lücke vor Eintreffen einer voraus-berechneten Wellenfront an der Erregungselektrode bei einer atrialen Fibrillation appliziert wird.

**Claims**

1.  A method for the predictive calculation of a cardiological signal curve, having the following method steps:

    - sampling the time curve of a cardiological signal during a specific sampling period,
    - calculating model coefficients according to the method of autoregressive modeling from the sampled values of the sampling period,

    **characterized by** a

    - predictive calculation of signal values to be expected following the sampling period during a prediction period on the basis of the sampled values and the model coefficients according to the method of autoregressive modeling, the signal values each being calculated iteratively by using the last previously calculated signal value as the last sampled value in the autoregressive modeling.

2.  The method according to Claim 1, **characterized in that** the cardiological signal is sampled using a cardiological device implant, in particular a cardiac pacemaker or defibrillator.

3.  The method according to Claim 1 or 2, **characterized in that** the excitation potential of the heart during the propagation of wavefronts on the atrial myocardium is sampled as the cardiological signal.

**4.** The method according to Claim 3, **characterized in that** the excitation potential is sampled using a bipolar electrode.

**5.** The method according to one of Claims 1 through 4, **characterized in that** autoregressive linear modeling is used as the model.

**6.** The method according to Claim 5, **characterized in that** the autoregressive modeling is performed according to the fundamental equation

$$y(n) = \sum_{j=1}^{M} d(j)\, y(n-j) + x(n)$$

with y (n): predictively calculated signal value,
d (j): one of M model coefficients in each case, and
x (n): permitted prediction error.

**7.** The method according to Claim 6, **characterized in that** the model coefficients d(j) are ascertained by determining the mean square prediction error over N >= M sampled values.

**8.** The method according to one of Claims 5 through 7, **characterized in that** a new set of model coefficients is determined not before the predictive calculation of the signal values in a new prediction period.

**9.** A cardiological device implant having

- a control unit,
- a measurement electrode,
- a computing unit for the predictive calculation of a cardiological signal curve according to one of Claims 1 through 8,
- a stimulation electrode, and
- a stimulation unit ensuring the electrical impingement of the stimulation electrode, which is operable according to a method for controlling the stimulation pulse delivery of a cardiological device implant, wherein, upon predictive calculation of a cardiological abnormal signal value curve with the aid of the prediction method according to one of Claims 1 through 8, a stimulation pulse counteracting the abnormal signal curve is delivered at an instant ascertained from the prediction or according to a method for controlling the stimulation pulse delivery of a cardiological device implant, the stimulation pulse being applied in the excitable gap before arrival of a predictively calculated wavefront at the excitation electrode during an atrial fibrillation.

**Revendications**

**1.** Procédé pour le calcul prévisionnel d'une courbe de signal cardiologique, comprenant les étapes de procédé suivantes :

- balayage de la variation dans le temps d'un signal cardiologique pendant une période de balayage définie,
- calcul de coefficients de modèle selon la méthode de modélisation autorégressive à partir des valeurs de balayage de la période de balayage, **caractérisé par** un
- calcul prévisionnel de valeurs de signal à escompter et consécutives à la période de balayage pendant une période de prévision sur la base des valeurs de balayage et des coefficients de modèle selon la méthode de modélisation autorégressive, les valeurs de signal étant calculées de façon itérative à chaque fois par l'utilisation de la dernière valeur calculée précédemment comme dernière valeur de balayage lors de la modélisation autorégressive.

**2.** Procédé selon la revendication 1, **caractérisé en ce que** le signal cardiologique est relié au moyen d'un implant d'appareil cardiologique, en particulier d'un pacemaker ou d'un défibrillateur.

**3.** Procédé selon la revendication 1 ou 2, **caractérisé en ce que,** comme signal cardiologique, le potentiel d'excitation du coeur est balayé lors de la propagation de fronts d'onde sur le myocarde atrial.

**4.** Procédé selon la revendication 3, **caractérisé en ce que** le potentiel d'excitation est balayé au moyen d'une électrode bipolaire.

**5.** Procédé selon l'une quelconque des revendications 1 à 4, **caractérisé en ce que** la modélisation linéaire autorégressive est utilisée comme modèle.

**6.** Procédé selon la revendication 5, **caractérisé en ce que** la modélisation autorégressive est effectuée selon l'équation de base

$$y(n) = \sum_{j=1}^{M} d(j)y(n-j) + x(n)$$

avec y(n) : valeur de signal précalculée
d(j) : à chaque fois l'un de M coefficients de modèle et
x(n) erreur de prévision autorisée

**7.** Procédé selon la revendication 6, **caractérisé en ce que** les coefficients de modèle d(j) sont déterminés par détermination de la valeur de prévision quadratique moyenne pour $N \geq M$ valeurs de balayage.

**8.** Procédé selon l'une quelconque des revendications 5 à 7, **caractérisé en ce qu'**un nouvel ensemble de coefficients de modèle n'est déterminé qu'avec le calcul préalable des valeurs de signal dans une nouvelle période de prévision.

**9.** Implant d'appareil cardiologique comprenant

- une unité de commande,
- une électrode de mesure,
- une unité de calcul pour le calcul prévisionnel d'une courbe de signal cardiologique selon l'une quelconque des revendications 1 à 8,
- une électrode de simulation et
- une unité de simulation garantissant l'alimentation électrique de l'électrode de simulation, laquelle unité peut être exploitée selon un procédé pour la commande et la génération d'impulsions d'un implant d'appareil cardiologique, une impulsion de stimulation s'opposant au tracé de signal anormal étant envoyée à un moment déterminé par la prévision en cas de calcul préalable d'une courbe de valeur de signal anormale au plan cardiologique à l'aide du procédé de prévision selon l'une quelconque des revendications 1 à 8, ou bien selon un procédé pour la commande de la génération d'impulsions de stimulation d'un implant d'appareil cardiologique, l'impulsion de stimulation étant appliquée lors de l'intervalle excitable avant l'arrivée d'un front d'onde précalculé sur l'électrode d'excitation dans le cas d'une fibrillation atriale.

FIG.1

FIG.2

FIG.3

FIG.4

FIG.5

FIG.6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0830876 A1

**In der Beschreibung aufgeführte Nicht-Patentliteratur**

- **THRONE R et al.** Autoregressive modeling of Epicardial Electrograms during Ventricular fibrillation. *IEEE Transaction Biomedical Engineering,* 01. April 1993, vol. 40 (4), 379-386